# FASCICULE DE BREVET EUROPEEN

(11) **EP 2 822 619 B1**
(45) Date de publication et mention de la délivrance du brevet: **09.05.2018**
(21) Numéro de dépôt: 13715276.5
(22) Date de dépôt: 07.03.2013
(51) Int. Cl.: A61M 5/315

(54) **SERINGUE POUR APPLICATION MÉDICALE**
SPRITZE ZUR MEDIZINISCHEN ANWENDUNG
SYRINGE FOR MEDICAL USE

(30) Priorité: 08.03.2012 FR 1252122
(43) Date de publication de la demande: 14.01.2015
(73) Titulaire: STEMCIS, 97490 Saint Clotilde (FR)
(72) Inventeur: ROCHE, Régis, 97417 La Montagne/Réunion (FR); FESTY, Franck, 97417 La Montagne/Réunion (FR)
(74) Mandataire: Jacobacci Coralis Harle
(86) Numéro de dépôt international: PCT/FR2013/050483
(87) Numéro de publication internationale: WO 2013/132192

(56) Documents cités:
- GB-A- 958 636
- GB-A- 1 225 495
- KR-A- 20060 046 868
- US-A- 5 531 691
- US-A- 5 713 914
- US-A- 5 891 052
- US-A1- 2011 009 829

## Description

L'invention concerne une seringue, et plus particulièrement une seringue pour application médicale.

L'invention concerne encore une seringue pouvant être utilisée lors d'opérations de lipoaspiration de tissus adipeux humains.

Les seringues sont depuis longtemps d'usage courant en médecine, pour des opérations soit de prélèvement (sanguin, tissulaire, *etc*...), soit d'injection (vaccins, tissulaire, *etc*...).

Les seringues sont notamment utilisées en chirurgie plastique dans les techniques de prélèvement et de réinjection de tissu adipeux, par exemple pour combler des pertes de substances sous-cutanées d'un patient. Différentes techniques de prélèvement de tissus adipeux sont aujourd'hui utilisées, par exemple l'excision ou la lipoaspiration, cette dernière pouvant être pratiquée à l'aide d'un système d'aspiration mécanique ou réalisée manuellement à l'aide d'une seringue.

Cette dernière technique utilisant une seringue comme moyen d'aspiration a été largement développée, mise au point et codifiée par S. R. Coleman, dans le but de réaliser une réinjection (greffe) de tissu adipeux qui soit pérenne (voir notamment l'article « Hand rejuvenation with structural fat grafting » in Plastic and Reconstructive Surgery, Vol. 110, No. 7, pp. 1731-1744, Décembre 2002 ou encore l'article « Structural Fat Grafting: more than a permanent filler » in Plastic and Reconstructive Surgery, Vol. 118, No. 3S, pp. 108S-120S, Septembre 2006).

Selon cette technique, il est préconisé de réaliser un prélèvement de tissu adipeux sur un patient de manière à traumatiser le moins possible les cellules graisseuses prélevées. Pour cela, il est nécessaire de contrôler précisément la dépression exercée par la seringue sur les tissus adipeux afin que celle-ci ne soit ni trop importante, ni trop brutale.

En pratique, le chirurgien utilisant cette technique prélève un premier volume de tissu adipeux avec la seringue en créant alors une dépression dans celle-ci en tirant le piston dans le corps de seringue. Il attend ensuite que les pressions dans la seringue et dans le corps du patient, à l'endroit du prélèvement, s'équilibrent, annulant ainsi la dépression à l'intérieur de la seringue. Le chirurgien peut alors prélever un deuxième volume de tissu, puis attendre, et renouveler autant de fois que nécessaire ces opérations jusqu'à prélever la quantité requise de tissu adipeux.

Par ailleurs, il a été suggéré que si la dépression appliquée par la seringue sur le tissu adipeux, lors de la remontée du piston de la seringue dans le corps de seringue, était trop importante, alors la qualité du prélèvement était moindre, compromettant ainsi la réussite des opérations suivant la lipoaspiration, notamment la survie à moyen et long terme du tissu adipeux après réinjection de celui-ci dans le patient prélevé.

Ainsi, il a été montré (*cf.* thèse de médecine de M. Ould Ali Djaffar, intitulée « Facteurs mécaniques influençant la qualité des transplants adipocytaires » délivrée par l'Université d'Aix-Marseille en 2010) que la dépression créée par la seringue devait rester inférieure à 0.4 atmosphère (atm) en valeur absolue, correspondant par exemple à un volume de dépression d'environ 2 centimètre cube (cc).

Le document US5047015 enseigne une seringue médicale avec un piston muni d'un filetage sur sa partie externe et d'un mécanisme de désengagement permettant de faire fonctionner le piston selon deux modes. Dans le premier mode, lorsque le mécanisme n'est pas activé, le filetage du piston s'engage et coopère avec un autre filetage présent sur la partie interne du corps de la seringue de telle sorte que le piston peut coulisser dans le corps de la seringue par rotation du piston dans le corps de seringue. Dans le deuxième mode, lorsque le mécanisme est activé, le filetage du piston se désengage de l'autre filetage de telle sorte que le piston peut coulisser librement dans le corps de la seringue

Le document US5215536 décrit une seringue médicale présentant un système de verrouillage permettant de maintenir le piston d'une seringue dans le corps de la seringue, sauf à pincer le corps de la seringue pour libérer le piston, celui-ci pouvant alors coulisser librement dans le corps de la seringue.

Le document GB 958636 enseigne une seringue destinée à délivrer une dose de substance calibrée, cette seringue comprenant un corps de seringue présentant un axe et un ressort maintenu dans le corps de seringue qui présente une élasticité dans un plan perpendiculaire à l'axe du corps de seringue, un piston comprenant une tige d'actionnement cruciforme avec quatre bras à 90° l'un de l'autre et des butées ménagées sur les faces axiales des bras adjacents de la croix de la tige d'actionnement.

Les butées sont réparties le long des faces axiales des bras de la tige d'actionnement de sorte que les butées sur les faces de deux bras situés dans un même plan sont alignées entre elles et décalées longitudinalement par rapport aux butées des deux autres bras à 90°.

Les butées sont conçues pour permettre, en coopération avec le ressort, une remontée, et une redescente, « cran à cran » du piston le long du corps de seringue.

Cependant, la seringue divulguée dans le document GB 958636 ne permet pas de désengager totalement les butées par un simple mouvement de rotation du piston dans le corps de seringue, celles-ci étant toujours en coopération avec le ressort.

Par ailleurs, l'utilisation d'une seringue selon l'art antérieur ne permet pas de contrôler précisément le volume de prélèvement, et donc la dépression exercée sur le tissu, lors de l'opération de lipoaspiration.

En effet, malgré les graduations souvent présentes sur la paroi extérieure du corps de seringue, il est difficile pour un chirurgien lors de l'opération de prélèvement de contrôler précisément ce volume de prélèvement, afin de ne pas dépasser la valeur limite de dépression indiquée ci-dessus.

De plus, un chirurgien utilisant une seringue selon l'art antérieur doit constamment garder son pouce sur le corps de la seringue afin de maintenir le piston en position dans le corps de seringue lors de l'étape d'équilibrage des pressions précédemment décrite. Une lipoaspiration manuelle utilisant une seringue de l'art antérieur est donc une opération pénible, car c'est le pouce du chirurgien qui sur l'ensemble de la durée de l'intervention fait l'effort de maintien du piston. Ceci induit de la fatigue pouvant conduire à des imprécisions des gestes du chirurgien ou à des erreurs de manipulation. De plus, lorsque cela devient douloureux pour le pouce, il existe un risque de « renoncement » de la part du chirurgien, qui aura tendance à augmenter la dépression exercée à chaque fois, afin d'écourter l'intervention.

On connaît par le brevet US-5.891.052 une seringue de prélèvement à préétablissement d'une dépression d'aspiration dans une chambre de dépression distincte de la chambre de prélèvement. La chambre de dépression est séparée de la chambre de prélèvement par un moyen d'étanchéité coulissant pouvant être immobilisé puis libéré. La dépression est réglable par blocage étagé de la tige du piston d'aspiration, la mobilité totale (tirage ou poussée) en coulissement de cette tige pouvant être obtenue par rotation de la tige supprimant le blocage étagé.

Ton connaît par le brevet US-5.531.691 une seringue pour injection à usage unique, avec indexation permettant de tirer ou pousser la tige de piston jusqu'à ce qu'à la fin de l'injection (poussée de la tige du piston), un blocage se produise. Une autre seringue d'aspiration est connue de KR20060046868. Afin de remédier à l'inconvénient précité de l'état de la technique, la présente invention propose une seringue permettant de contrôler la dépression appliquée par la seringue lors de la remontée du piston et de maintenir sans effort le piston dans sa position à l'intérieur du corps de la seringue.

À cet effet, l'invention concerne une seringue selon la revendication 1. Ainsi, la seringue selon l'invention permet de réaliser des prélèvements de tissus adipeux par lipoaspiration, en contrôlant précisément la position du piston dans la seringue et donc le volume de tissu prélevé grâce aux butées et aux crans en saillie, et donc de s'assurer que la dépression appliquée par la seringue est inférieure à la limite préconisée. Le chirurgien ne risque donc pas d'augmenter ou de diminuer le volume de prélèvement, et par la même la dépression exercée sur les tissus adipeux.

De plus, les crans en saillie et les butées coopèrent de manière à maintenir le piston dans sa dernière position en l'empêchant de redescendre.

En empêchant le piston de redescendre dans le corps de seringue par simple pression, la seringue selon l'invention permet de libérer le pouce du chirurgien réalisant l'opération, pouce qui n'a alors plus besoin de faire d'effort de maintien du piston dans le corps de seringue. Ainsi, le pouce est moins fatigué et les gestes du chirurgien plus précis.

Cela permet d'autre part d'éviter de réinjecter par erreur du tissu adipeux venant juste d'être prélevé. Car la seringue selon l'invention n'est pas prévue pour des injections mais pour des aspirations.

Selon l'invention, il est nécessaire de désengager la première crémaillère par rotation du piston et amener volontairement la première crémaillère en regard des surfaces vierges si l'on veut que celui-ci redescende.

De plus, lorsque cela est nécessaire, la surpression créée par la redescente du piston se doit d'être la plus faible possible afin de traumatiser le moins possible les cellules graisseuses prélevées.

Enfin, la seringue selon l'invention, lorsqu'elle est mise dans une centrifugeuse après prélèvement, empêche une redescente du piston dans le corps de seringue sous l'effet de la force centrifuge, évitant ainsi une dégradation des cellules adipeuses prélevées.

Par ailleurs, d'autres caractéristiques avantageuses et non limitatives de la seringue selon l'invention sont les suivantes :
- le corps de seringue comporte une deuxième butée semblable à la première butée, et située le long de la paroi interne du corps de seringue à proximité de l'ouverture supérieure, de sorte que la première et la deuxième butées forment entre elles une première et une deuxième surfaces vierges sur la paroi interne du corps de seringue, et la tige d'actionnement comporte sur sa surface externe une deuxième crémaillère semblable à la première crémaillère et disposée longitudinalement le long de la tige d'actionnement, de sorte que, lorsque la première crémaillère est en regard de la première butée, la deuxième crémaillère est en regard de la deuxième butée ;
- les première et deuxième butées sont sensiblement diamétralement opposées, et les première et deuxième crémaillères sont également sensiblement diamétralement opposées.

Ainsi, la seringue selon l'invention qui possède au moins deux butées, de préférence diamétralement opposées permet de limiter les mouvements transversaux du piston à l'intérieur du corps de seringue et rend plus aisée sa manipulation lors de l'opération de lipoaspiration. De plus, le désengagement des deux crémaillères devant être simultané, la redescente du piston dans le corps de seringue par erreur est rendu plus difficile.

D'autres caractéristiques avantageuses et non limitatives de la seringue selon l'invention sont également les suivantes :
- la tête de piston comporte une partie terminale faisant face à l'extrémité inférieure du corps de seringue, la partie terminale étant telle qu'elle empêche tout désengagement complet du piston du corps de seringue par blocage par la ou les butées, et une embase, raccordée à la partie terminale comprenant à sa périphérie au moins une première encoche et au moins une première rainure longitudinale, la première encoche et la première rainure longitudinale de l'embase étant conformées et constituées pour permettre la remontée et le maintien du piston en position haute lorsque la première encoche est en regard avec la première butée, et empêcher toute redescente du piston le long du corps de seringue, sauf à désengager la tête de piston par rotation du piston dans le corps de seringue autour de l'axe longitudinal pour amener la première rainure longitudinale en regard de la première butée ;
- l'embase comprend à sa périphérie une deuxième encoche semblable à la première encoche, et une deuxième rainure longitudinale semblable à la première rainure longitudinale, disposée de telle sorte que, lorsque la première rainure longitudinale est en regard de la première butée, la deuxième rainure longitudinale est en regard de la deuxième butée ;
- les première et deuxième rainures longitudinales sont sensiblement diamétralement opposées
- la tige d'actionnement est détachable de la tête de piston ;
- la tête de piston comporte un logement de réception solidaire de l'embase de sorte que, lorsque le piston est en position haute, le logement de réception se trouve hors du corps de seringue, et la tige d'actionnement du piston comporte à l'une de ses extrémités un élément de raccordement à la tête de piston, l'élément de raccordement présentant une forme complémentaire du logement de réception de la tête de piston, de sorte que, lorsque le piston est en position haute, l'élément de raccordement peut s'engager ou se désengager du logement de réception et que, lorsque l'élément de raccordement est engagé à l'intérieur du logement de réception, la tige d'actionnement et la tête de piston sont solidaires en translation selon l'axe longitudinal et en rotation autour de l'axe longitudinal ;
- le logement de réception comprend une face latérale ouverte et une face supérieure présentant une ouverture centrale sensiblement positionnée selon l'axe longitudinal de la seringue, de telle sorte que l'élément de raccordement s'engage ou se désengage du logement de réception par glissement au travers de la face latérale ouverte, l'ouverture centrale permettant le passage de la tige d'actionnement.

La seringue selon l'invention est particulièrement bien adaptée à la mise en oeuvre de cette seringue dans une centrifugeuse, après l'opération de prélèvement.

En effet, une fois le prélèvement terminé et la seringue retirée du corps du patient, il est parfois nécessaire de séparer les différentes cellules constituant le tissu adipeux prélevé par centrifugation. À ce stade, le piston de la seringue est généralement dans sa position haute, si bien que la seringue dans son ensemble présente un encombrement maximal. Cependant, l'espace disponible dans une centrifugeuse est souvent limité, si bien que le détachement de la tige d'actionnement de la tête de piston permet de réduire la taille totale de la seringue pour la rentrer dans la centrifugeuse.

Par ailleurs, selon l'invention, la tête de piston peut être maintenue en position haute, même une fois que la tige d'actionnement est détachée de la tête de piston. Ainsi, lors de la centrifugation, il n'y a pas de risque ni que le piston ne rentre à nouveau dans le corps de seringue jusqu'à une position basse, ni que le piston ne se désengage complètement du corps de seringue, ce qui serait extrêmement dommageable pour les tissus prélevés.

Un mode de réalisation particulier de l'invention sera décrit en détail ci-après en référence aux dessins annexés dans lesquels :
- la figure 1 est une vue d'ensemble d'une seringue selon un mode de réalisation de l'invention ;
- la figure 2 est une vue en coupe longitudinale du corps d'une seringue selon le mode de réalisation de la figure 1 ;
- la figure 3 est une vue de dessus du corps d'une seringue selon le mode de réalisation de la figure 1 ;
- la figure 4 est une vue en perspective d'une bague comprenant deux butées en regard l'une de l'autre ;
- la figure 5 est une vue en coupe longitudinale d'une tige d'actionnement d'un piston de seringue selon le mode de réalisation de l'invention de la figure 1 ;
- la figure 6 une vue en coupe transverse (selon la direction A-A de la figure 5) de la tige d'actionnement de piston telle que représentée sur la figure 5 ;
- la figure 7 est une vue en coupe longitudinale d'une tête de piston de seringue selon le mode de réalisation de l'invention de la figure 1 ;
- la figure 8 une vue en coupe transverse (selon la direction B-B de la figure 7) de la tête de piston telle que représentée sur la figure 7 ;
- la figure 9 est une vue en perspective d'un logement de réception selon le mode de réalisation de l'invention de la figure 1.
- la figure 10 est une vue en coupe d'une seringue selon le mode de réalisation de la figure 1, lorsque le piston est en position basse.
- la figure 11 est une vue en coupe d'une seringue selon le mode de réalisation de la figure 1, lorsque le piston est dans une position intermédiaire.
- la figure 12 est une vue en coupe d'une seringue selon le mode de réalisation de la figure 1, lorsque le piston est en position haute ;
- la figure 13 est une vue en coupe d'une seringue selon le mode de réalisation de la figure 1, lorsque la tête de piston est bloquée par les première et deuxième butées.

En préambule, précisons que dans l'exposé qui va suivre, les termes « haut(e) » ou « supérieur(e) » et « bas(se) » ou « inférieur(e)» seront utilisés relativement à la seringue, le bas désignant le côté fixé à l'aiguille ou à la canule et le haut le côté duquel dépasse le piston de la seringue. De même, on considérera que la seringue s'étend le long d'une direction sensiblement verticale.

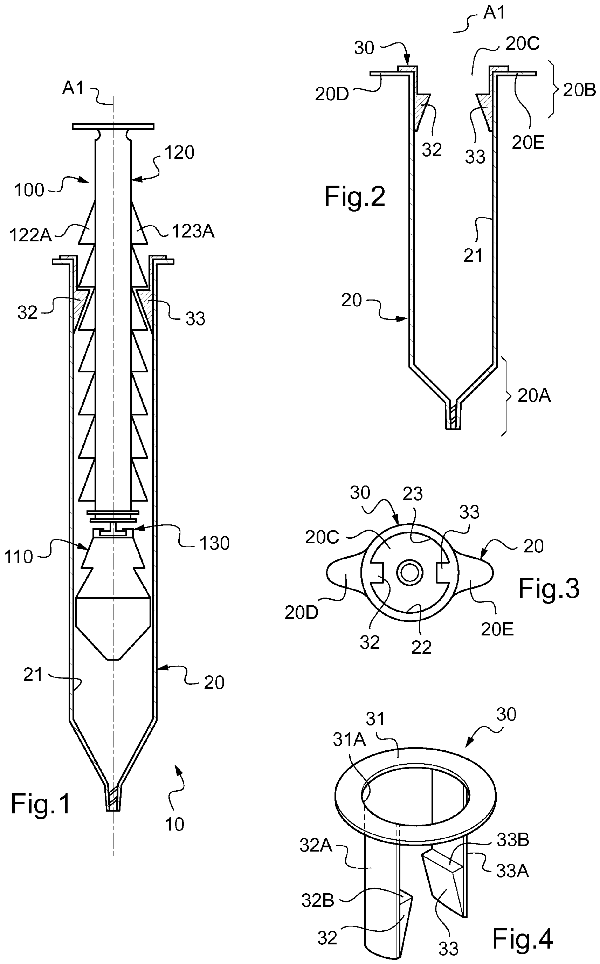

La figure 1 représente une vue d'ensemble d'une seringue 10 selon un mode de réalisation particulier de l'invention, cette seringue comportant un corps de seringue 20.

Comme représenté sur la figure 2, le corps de seringue 20 présente dans sa partie intérieure une paroi interne 21.

Le corps de seringue 20 comprend une extrémité inférieure 20A servant à la fixation d'une aiguille ou d'une canule. Cette extrémité inférieure 20A est ici de forme tronconique. Le corps de seringue 20 comprend également une extrémité supérieure 20B présentant une ouverture circulaire 20C et deux zones plates 20D, 20E. Cette ouverture circulaire 20C permet l'introduction du piston 100 comme décrit en détails ci-après.

Le corps de seringue 20 est sensiblement de révolution autour d'un axe longitudinal A1, si bien que la partie intermédiaire du corps de seringue, située entre l'extrémité inférieure 20A et l'extrémité supérieure 20B est cylindrique.

Le corps de seringue 20 présente ici une hauteur totale de l'ordre de 80 millimètres et un diamètre intérieur de l'ordre de 15 millimètres, définissant ainsi un volume d'environ 14 millilitres.

En outre, le corps de seringue 20 présente ici une épaisseur d'environ 1 millimètre.

En variante, le corps de seringue peut par exemple présenter une hauteur totale allant jusqu'à 150 millimètres et un diamètre intérieur de 25 millimètres, définissant ainsi un volume pouvant aller jusqu'à 75 millilitres environ.

Selon ce mode de réalisation, le corps de seringue 20 est réalisé en polypropylène par moulage.

En variante, le corps de seringue peut être réalisé dans une autre matière plastique comme par exemple le polycarbonate, en métal ou bien en verre.

Le corps de seringue 20 est de préférence transparent. Ceci permet au chirurgien effectuant un prélèvement avec une seringue 10 selon le mode de réalisation de l'invention de voir à l'intérieur de celle-ci la quantité et éventuellement la nature des tissus prélevés.

Le corps de seringue 20 est muni à proximité de son extrémité supérieure 20B d'une pièce en forme de bague 30.

Comme représenté sur la figure 4, cette bague 30 comporte une partie annulaire plate 31 ainsi que deux bras 32A, 33A s'étendant vers le bas à partir de la face inférieure de la partie annulaire plate 31 depuis son bord intérieur 31A. Selon le mode de réalisation de l'invention, les deux bras 32A, 33A sont ici diamétralement opposés.

Le bras 32A comporte une première butée 32 et le bras 33A comporte une deuxième butée 33, les première et deuxième butées 32, 33 étant donc diamétralement opposées. Les première et deuxième butées 32, 33 présente une forme de prisme triangulaire dont la pointe est orientée vers le bas, et sont saillantes l'une vers l'autre.

Les première et deuxième butées 32, 33 présentent des surfaces d'appui 32B et 33B sensiblement horizontales et de forme rectangulaire.

Les première et deuxième butées 32, 33 ont ici une hauteur comprise entre 2 et 15 millimètres et les surfaces d'appui 32B, 33B ont une largeur comprise entre 1 et 10 millimètres et une profondeur comprise entre 0.5 et 7 millimètres.

Selon ce mode de réalisation, la bague 30 est réalisée en polypropylène, par moulage. De manière préférentielle, la bague 30 est constituée d'un matériau de couleur différente du corps de seringue 20 afin de pouvoir les distinguer et s'assurer rapidement de la présence de la bague 30 dans le corps de seringue 20.

En variante, la bague peut être réalisée dans une autre matière plastique comme par exemple le polycarbonate, ou encore en métal.

Selon le mode de réalisation de l'invention décrit ici, la bague 30 est insérée et fixée dans le corps de seringue 20 par emboîtement de la bague 30 au niveau de l'ouverture circulaire 20C, le matériau de la bague 30 étant suffisamment souple pour se déformer élastiquement et rentrer « en force » dans le corps de seringue 20.

Comme représenté sur les figures 2 et 3, la partie annulaire plate 31 vient alors en appui sur les deux zones plates 20D, 20E et sur le bord circulaire de l'extrémité supérieure 20B du corps de seringue 20. De même, les dimensions de la bague 30 sont ajustées pour que la distance entre les deux bras 32A, 33A soit très légèrement supérieure au diamètre intérieur du corps de seringue 20, les bras 32A, 33A venant alors s'appuyer sur la paroi interne 21 du corps de seringue 20, si bien que le désemboîtement de la bague 30 du corps de seringue 20 ou sa rotation à l'intérieur du corps de seringue 20 selon l'axe longitudinal A1 requiert un effort important.

Ainsi agencés, la bague 30 et le corps de seringue 20 laissent entre les première et deuxième butées 32, 33 en regard, une première et une deuxième surfaces vierges 22, 23 sur la paroi interne 21 du corps de seringue 20. Les première et deuxième butées 32, 33 étant ici sensiblement diamétralement opposées, les première et deuxième surfaces vierges 22, 23 sont donc également sensiblement diamétralement opposées.

Comme représenté sur la figure 1, la seringue 10 selon ce mode de réalisation de l'invention comporte également un piston 100, le piston 100 comprenant une tête de piston 110, une tige d'actionnement 120 et un logement de réception 130.

La longueur totale de la tige d'actionnement 120 est ici d'environ 100 millimètres.

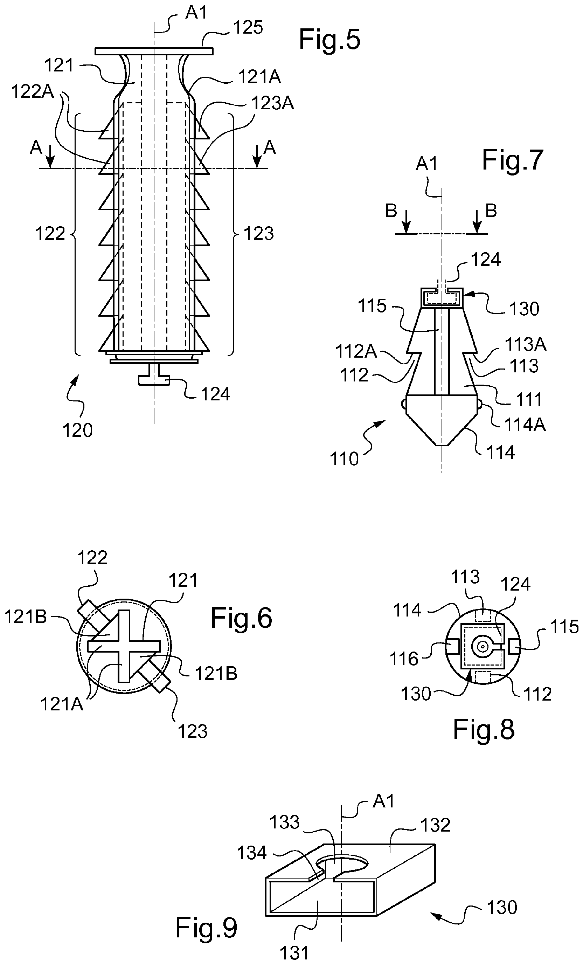

La tige d'actionnement 120 comporte une surface externe 121 dont la structure précise est décrite sur les figures 5 et 6.

Cette surface externe 121 comprend un croisillon 121A avec quatre bras deux à deux orthogonaux et deux éléments de support 121B disposés dans deux intervalles opposés, formés entre deux bras consécutifs du croisillon 121A.

Les deux éléments de support 121B s'étendent longitudinalement le long de la tige d'actionnement 120. L'un des éléments de support 121B comporte une première crémaillère 122 disposée longitudinalement le long de la tige d'actionnement 120, la première crémaillère 122 comprenant des crans en saillie 122A.

L'autre élément de support 121B comporte une deuxième crémaillère 123 semblable à la première crémaillère 122 comprenant des crans en saillie 123A et disposée longitudinalement le long de la tige d'actionnement 120.

Ces crans en saillie 122A, 123A sont saillants vers l'extérieur à partir de la surface externe 121 de la tige d'actionnement 121. Comme représenté sur le figure 6, les crans en saillie 122A, 123A sont plus précisément positionnés sur deux branches opposées de la surface externe 121 en forme de croix.

Les crans en saillie 122A, 123A sont en forme de prisme triangulaire, d'orientation inversée par rapport aux butées 32, 33. Ils sont réalisés dans une matière plastique souple.

Selon ce mode de réalisation, les première et deuxième crémaillères 122, 123 sont ici diamétralement opposées et comprennent chacune sept crans en saillies 122A, 123A, de préférence équidistants les uns des autres, les crans en saillie 123A de la deuxième crémaillère 123 étant diamétralement opposés aux crans en saillie 122A de la première crémaillère 122.

La tige d'actionnement 120 comporte dans sa partie inférieure un élément de raccordement 124 comportant une partie parallélépipédique surmontée d'une partie cylindrique. Le fonctionnement de cet élément de raccordement 124 sera décrit plus en détails par la suite, notamment lors de la description du logement de réception 130.

La tige d'actionnement 120 comporte par ailleurs dans sa partie supérieure une zone aplatie 125 permettant de faire coulisser le piston 100 dans le corps de seringue 20, soit vers le bas en poussant sur la zone aplatie 125, soit vers le haut en tirant sur la zone aplatie 125.

Le piston 100 comprend également une tête de piston 110 telle que décrite sur les figures 7 et 8.

La tête de piston 110 comporte à son extrémité inférieure une partie terminale 114 qui est de révolution autour de l'axe longitudinal A1. La partie terminale 114 est une partie terminale classique d'une tête de piston de seringue. Elle a une forme cylindrique dans sa partie haute pour s'adapter à la forme cylindrique de la partie intermédiaire du corps de seringue 20 et une forme conique dans sa partie basse afin de s'adapter à la forme tronconique de l'extrémité inférieure 20A du corps de seringue 20.

La partie terminale 114 comporte un joint torique 114A à sa périphérie de sorte que le piston 100 coulisse de manière étanche le long de la paroi interne 21 du corps de seringue 20, empêchant ainsi toute fuite de la substance qui serait prélevé ou injecté avec la seringue 10.

La partie terminale 114 de la tête de piston 110 est réalisé en polypropylène.

En variante, la partie terminale peut être réalisé en polyéthylène, en polytétrafluoroéthylène (PTFE), ou en perfluoro-éthylène-propylène (*« fluorinated ethylene propylene »* ou FEP en anglais).

La partie terminale 114 est généralement de couleur noire afin de faciliter la lecture du volume prélevé ou injecté sur les graduations présentes sur le corps de seringue 20.

Comme représenté sur la figure 7, sur la face supérieure de la partie terminale 114 de la tête de piston 110 est fixée une embase 111. Cette embase 111 est de forme tronconique.

La base du cône qui est liée à la partie terminale 114 a un diamètre compris entre 14 et 25 millimètres selon le modèle de seringue considéré. Dans sa partie haute, l'embase 111 présente une section conique de diamètre comprise entre 12 et 23 millimètres.

L'embase 111 comporte une première encoche 112 et une deuxième encoche 113 en creux pratiquées chacune à sa périphérie, de telle sorte que les première et deuxième encoches 112, 113 sont diamétralement opposées, comme cela est visible sur la figure 8. Les première et deuxième encoches 112, 113 sont situées environ à mi-hauteur de l'embase 111, séparant l'embase en une partie haute et une partie basse.

Les première et deuxième encoches 112, 113 sont de forme triangulaire de manière à définir une première et une deuxième faces planes 112A, 113A sensiblement horizontale tournée vers le bas.

Dans le mode de réalisation particulier décrit sur les figures 7 et 8, les première et deuxième encoches 112, 113 ont une profondeur comprise entre 0.5 et 7 millimètres et une largeur comprise entre 1 et 10 millimètres.

Ces dimensions sont sensiblement identiques aux dimensions des première et deuxième butées 32, 33 de telle sorte que les première et deuxième butées 32, 33 soient de forme sensiblement complémentaires des première et deuxième encoches 112, 113.

L'embase 111 comporte également une première rainure 115 et une deuxième rainure 116 rectilignes, pratiquées sur sa périphérie, sur toute la hauteur de l'embase 111.

Comme représenté sur la figure 8, les première et une deuxième rainures rectilignes 115, 116 sont diamétralement opposées et disposées sur la périphérie de l'embase 111 de telle sorte que, d'une part, la première rainure rectiligne 115 est située entre la première encoche 112 et la deuxième encoche 113, et forme avec chacune d'elles un angle de 90 degrés et que, d'autre part, la deuxième rainure rectiligne 116 est située entre la deuxième encoche 113 et la première encoche 112, et forme avec chacune d'elles également un angle de 90 degrés.

En variante, les première et deuxième encoches 112, 113 et les première et deuxième rainures rectilignes 115, 116 peuvent ne pas être diamétralement opposées. De même, les première et deuxième encoches 112, 113 et les première et deuxième rainures rectilignes 115, 116 peuvent être séparées par un angle différent de 90 degrés.

Les première et deuxième rainures rectilignes 115, 116 présentent une largeur comprise entre 1 et 11 millimètres et une profondeur comprise entre 0.5 et 8 millimètres. Leur largeur est légèrement supérieure à la largeur des première et deuxième butées 32, 33.

L'embase 111 est réalisée dans un matériau plastique souple de type polyéthylène.

En variante, l'embase peut être par exemple réalisée en polypropylène, en polycarbonate, en polytétrafluoroéthylène, ou en perfluoro-éthylène-propylène.

La tête de piston 110 comporte enfin à son extrémité supérieure un logement de réception 130 fait par usinage.

Alternativement, le logement de réception est fixé sur la partie supérieure de l'embase 111 par collage.

Comme illustré sur la figure 9, ce logement de réception 130 est de forme parallélépipédique. Il définit ainsi un volume de réception. Ici, le logement de réception 130 est à base carrée: il présente une arête comprise entre 10 et 30 millimètres et une hauteur comprise entre 5 et 15 millimètres suivant la taille de la seringue considérée.

Le logement de réception 130 comprend une face latérale ouverte 131 et une face supérieure 132 perpendiculaire à l'axe longitudinal A1.

Cette face supérieure 132 possède en son centre une ouverture circulaire 133 centrée selon l'axe longitudinal A1 et une rainure droite 134 s'étendant depuis la face latérale ouverte 131 jusqu'à l'ouverture centrale 133.

Le logement de réception 130 est ainsi dimensionné pour recevoir l'élément de raccordement 124 de la tige d'actionnement 120.

La tige d'actionnement 120 est ici détachable de la tête de piston 110 grâce à la liaison amovible entre l'élément de raccordement 124 et le logement de réception 130.

En effet, l'élément de raccordement 124 s'engage ou se désengage du logement de réception 130 par glissement au travers de la face latérale ouverte 131, la partie parallélépipédique de l'élément de raccordement 124 venant s'emboîter ou se désemboîter du logement de réception 130, la rainure droite 134 et l'ouverture centrale 133 permettant le passage de la partie cylindrique de l'élément de raccordement 124 lors de l'emboîtement ou du désemboîtement de la partie parallélépipédique de l'élément de raccordement 124.

Une fois engagé à l'intérieur du logement de réception 130, l'élément de raccordement 124 ne peut plus tourner dans le logement de réception 130. Dans ce cas, l'élément de raccordement 124 étant attaché à la tige d'actionnement 120 d'une part et le logement de réception 130 étant attaché à la tête de piston 110 d'autre part, la tige d'actionnement 120 est solidaire en rotation autour de l'axe longitudinal A1 de la tête de piston 110.

De la même manière, la tige d'actionnement 120 est solidaire en translation de la tête de piston 110 le long de l'axe longitudinal A1.

Lorsque la tige d'actionnement 120 est raccordée à la tête de piston 110 au niveau de son extrémité supérieure, l'encoche 112 et la crémaillère 122 d'un côté, et l'encoche 113 et la crémaillère 123 d'un autre côté, sont ici alignées.

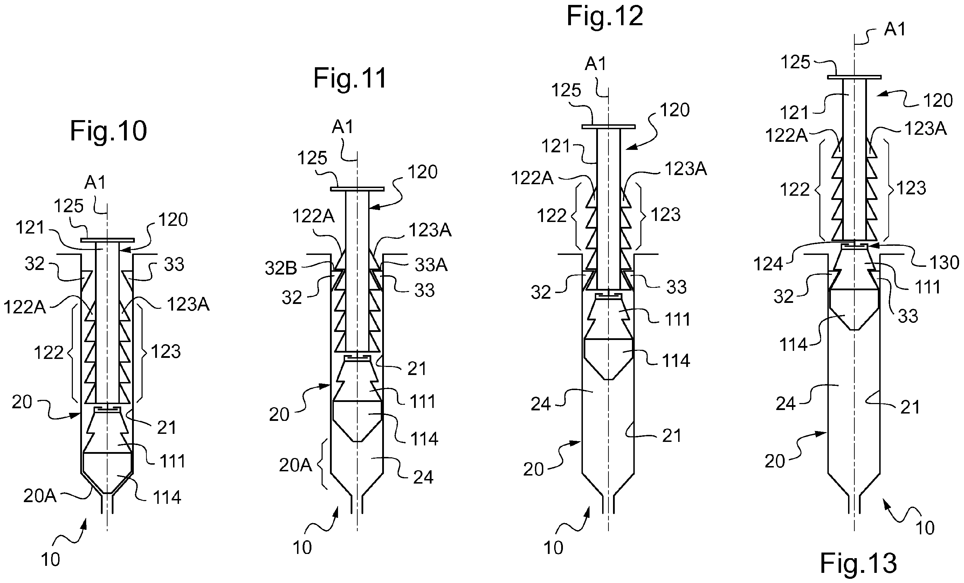

Le fonctionnement de la seringue 10 selon le mode de réalisation particulier décrit ci-dessus sera maintenant décrit par rapport aux figures 10 à 13.

On considère ici une opération de prélèvement de tissu effectué par un opérateur où l'état initial de la seringue 10 est celui représenté sur la figure 10.

Dans cette position initiale, le piston 100 est dans sa position basse par rapport au corps de seringue 20, c'est-à-dire que la partie terminale 114 de la tête de piston 110 appuie sur l'extrémité inférieure 20A du corps de seringue 20, leur forme respective étant complémentaire l'une de l'autre.

La tige d'actionnement 120 est en partie à l'intérieur du corps de seringue 20, son extrémité supérieure et notamment la zone aplatie 125 étant situées à l'extérieur du corps de seringue permettant à l'opérateur manipulant la seringue 10 de faire coulisser le piston 100 le long de la paroi interne 21 du corps de seringue 20 suivant l'axe longitudinal A1, en tirant sur la tige d'actionnement au niveau de la zone aplatie 125.

Dans la situation illustrée sur la figure 10, la première crémaillère 122 est alignée avec la première butée 32, et la deuxième crémaillère 123 est alignée avec la deuxième butée 33. La largeur de la tige d'actionnement 120 au niveau de la surface externe 121 comportant les première et deuxième crémaillères 122, 123 est inférieure au diamètre intérieur du corps de seringue 20 de telle sorte que les crans en saillies 122A, 123A ne frottent pas sur la paroi interne 21 du corps de seringue 20, limitant ainsi la force de traction que l'opérateur doit exercer sur la tige d'actionnement 120 pour faire coulisser le piston 100 dans le corps de seringue 20 suivant l'axe longitudinal A1.

À partir de la situation illustrée sur la figure 10, l'opérateur tire donc sur la tige d'actionnement 120 pour commencer à prélever du tissu. Le piston 100 coulisse alors dans le corps de seringue 20 suivant l'axe longitudinal A1. Les première et deuxième crémaillères 122, 123 et plus particulièrement les deux premiers crans en saillie 122A, 123A tournées vers l'extrémité supérieure de la tige d'actionnement 120 montent à l'intérieur du corps de seringue 20 jusqu'à être en face des première et deuxième butées 32, 33.

La distance entre les deux premiers crans en saillie 122A, 123A étant supérieure à la distance entre les première et deuxième butées 32, 33, l'opérateur ne peut continuer à tirer facilement sur la tige d'actionnement 120.

L'opérateur doit alors exercer une force supplémentaire afin de faire passer les deux premiers crans en saillie 122A, 123A au-delà des première et deuxième butées 32, 33 grâce à la déformation élastique des deux premiers crans en saillie 122A, 123A.

Une fois les première et deuxième butées 32, 33 passées, les deux premiers crans en saillie 122A, 123A reposent sur les surfaces d'appui 32B, 33B des première et deuxième butées 32, 33 empêchant toute redescente du piston 100 par pression sur la tige d'actionnement 120.

Dans cette configuration représentée sur la figure 11, la partie terminale 114 de la tête de piston 110 ne repose plus sur l'extrémité inférieure 20A du corps de seringue 20 mais est remontée à l'intérieur du corps de seringue 20, définissant ainsi un premier volume de prélèvement 24.

La position des deux premiers crans en saillie 122A, 123A sont déterminés pour que ce premier volume de prélèvement 24 corresponde à un volume tel que la dépression correspondante exercée sur les tissus prélevés soit inférieure à une valeur seuil.

Ici, le corps de seringue 20 ayant un volume total de 10 millilitres, le premier volume de prélèvement 24 est égal à 2 centimètre cube, limitant la dépression exercée à une valeur de 0.4 atmosphère (en valeur absolue).

En répétant plusieurs fois l'étape précédente, l'opérateur remonte le piston 100 « cran par cran » dans le corps de seringue 20, le volume supplémentaire prélevé, et donc la dépression exercée sur les tissus prélevés, étant toujours le même, du fait de l'écart constant déterminé entre les crans en saillie 122A, 123A.

La figure 12 représente une situation intermédiaire où le piston 100 est situé entre la position basse précédemment décrite et la position haute décrite plus en détails par la suite. Dans cette configuration, les première et deuxième butées 32, 33 et les première et deuxième crémaillères 122, 123 sont en regard chacune l'une de l'autre. Deux crans en saillie 122A, 123A reposant sur les deux surfaces d'appuis 32B, 33B des butées 32, 33, le piston 100 ne peut redescendre dans le corps de seringue 20.

Afin de désengager la première et la deuxième crémaillère 122, 123, l'opérateur amène la première crémaillère 122, respectivement la deuxième crémaillère 123, en regard de la première surface vierge 22, respectivement de la deuxième surface vierge 23, de la paroi interne 21 du corps de seringue 20 par rotation du piston 100 dans le corps de seringue 20 autour de l'axe longitudinal A1.

Ainsi, la largeur de la surface externe 121 de la tige d'actionnement 120 étant inférieure à la distance entre la première et la deuxième butée 32, 33, le piston 100 peut coulisser sans entrave le long de la paroi interne 21 du corps de seringue 20.

L'opérateur peut alors soit amener le piston 100 jusqu'à sa position basse, telle que représentée sur la figure 10, ou bien bloquer de nouveau le piston 100 en position intermédiaire en réengageant la première et la deuxième crémaillères 122, 123 dans les première et deuxième butées 32, 33 par rotation de la tige d'actionnement 120.

Si à partir de la situation représentée sur la figure 12, l'opérateur continue de remonter cran à cran le piston 100 en le faisant coulisser le long du corps de seringue 20, il finira par faire passer tous les crans en saillie 122A, 123A des première et deuxième crémaillères 122, 123. Seule la tête de piston 110 demeure alors à l'intérieur du corps de seringue 20, et notamment le logement de réception 130, interdisant ainsi tout détachement de la tige d'actionnement 120 de la tête de piston 110 par glissement de l'élément de raccordement 124 hors du logement de réception 130.

En continuant d'exercer une force de traction sur la tige d'actionnement 120, l'opérateur fait passer la partie haute de l'embase 111 entre les première et deuxième butées 32, 33, l'embase 111 se déformant grâce à son élasticité, comme les crans en saillie 122A, 123A. Les première et deuxième butées 32, 33 sont alors en regard des première et deuxième encoches 112, 113, et les première et deuxième faces planes 112A, 113A des première et deuxième encoches 112, 113 reposent sur les première et deuxième surfaces d'appui 32B, 33B des première et deuxième butées 32, 33.

Comme précédemment, le passage de la première partie de l'embase 111 augmente le volume de prélèvement d'une valeur telle que la dépression correspondante exercée sur les tissus prélevés est inférieure à la valeur seuil précédemment définie, et de préférence analogue au passage cran à cran.

Lorsque les première et deuxième encoches 112, 113 sont en regard avec les première et deuxième surfaces d'appui 32B, 33B des première et deuxième butées 32, 33, le piston 100 est maintenu en position haute et ne peut redescendre le long du corps de seringue 20.

Afin de désengager la tête de piston 110, l'opérateur amène la première rainure longitudinale 115 en regard de la première butée 32 par rotation du piston 100 dans le corps de seringue 20 au moyen de la tige d'actionnement 120. La deuxième butée 33 et la deuxième rainure longitudinale 116 étant respectivement diamétralement opposées à la première butée 32 et à la première rainure longitudinale 115, la deuxième butée 33 et la deuxième rainure longitudinale 116 se retrouve alors également en regard.

De plus, les première et deuxième crémaillères 122, 123 étant alignées avec les première et deuxième encoches 112, 113, elles se trouvent au-dessus et dans l'alignement des première et deuxième surfaces vierges 22, 23.

Ainsi, l'opérateur peut faire coulisser vers le bas le piston 100 à l'intérieur du corps de seringue 20 en exerçant une force de pression sur la tige d'actionnement 120.

Par ailleurs, comme représenté sur la figure 13, lorsque le piston 100 est en position haute, la partie terminale 114 empêche tout désengagement complet du piston 100 du corps de seringue 20 par blocage de la tête de piston 110 par la première et la deuxième butées 32, 33.

En effet, la partie terminale 114 étant constituée d'une matière rigide, l'opérateur ne peut désengager complètement le piston 100 du corps de seringue 20 sauf à exercer volontairement une force de traction très importante sur la tige d'actionnement 120. Ceci évite que des erreurs de manipulation involontaires désengagent complètement la tête de piston 110 du corps de seringue 20 et compromettent éventuellement la qualité du prélèvement.

En outre, la position longitudinale des première et deuxième encoches 112, 113 sur l'embase 111 est telle que, dans la position haute représentée sur la figure 13, l'extrémité supérieure de la tête de piston, et plus particulièrement le logement de réception 130, dépasse suffisamment l'extrémité supérieure 20B du corps de seringue 20 afin de permettre le désengagement de l'élément de raccordement 124 par son glissement hors du logement de réception 130.

La tige d'actionnement 120 peut ainsi être détachée de la tête de piston 110, réduisant la longueur totale de la seringue 10 lorsque la tête de piston 110 est en position haute et facilitant ainsi la manipulation de la seringue 10.

## Revendications

1. Seringue (10) comportant :
- un corps de seringue (20) s'étendant suivant un axe longitudinal (A1) d'une extrémité inférieure (20A) à une extrémité supérieure (20B), l'extrémité inférieure (20A) du corps de seringue (20) permettant la fixation d'une aiguille ou d'une canule et l'extrémité supérieure (20B) du corps de seringue (20) présentant une ouverture supérieure (20C) permettant l'introduction d'un piston dans le corps de seringue (20), le corps de seringue (20) présentant une paroi interne (21) et comportant au moins une première butée (32) située le long de la paroi interne (21) du corps de seringue (20) à proximité de l'ouverture supérieure (20C), la première butée (32) étant conçue pour laisser au moins une première surface vierge (22) sur la paroi interne (21) du corps de seringue (20), et
- un piston (100) coulissant le long de la paroi interne (21) du corps de seringue (20) suivant l'axe longitudinal (A1), le piston (100) comprenant une tête de piston (110) et une tige d'actionnement (120) raccordée à l'extrémité supérieure (110B) de la tête de piston (110), l'extrémité supérieure (110B) de la tête de piston (110) étant tournée vers l'ouverture supérieure (20C) du corps de seringue (20), la tige d'actionnement (120) du piston (100) comporte, sur sa surface externe (121), au moins une première crémaillère (122) disposée longitudinalement le long de la tige d'actionnement (120), la première crémaillère (122) comprenant des crans en saillie (122A),
la première butée (32) et les crans en saillie (122A) étant conformés et constitués pour, lors de la remontée du piston (100) dans le corps de seringue (20) d'une position basse où la tête de piston (110) est à proximité de l'extrémité inférieure (20A) du corps de seringue (20) à une position haute où la tête de piston (110) est à proximité de l'extrémité supérieure (20B) du corps de seringue (20), lorsque la première butée (32) et la première crémaillère (122) sont en regard l'une de l'autre, permettre au piston (100) une remontée cran par cran le long du corps de seringue (20),
la seringue (10) étant **caractérisée en ce que** la première butée (32) et les crans en saillie (122A) sont également conformés et constitués pour empêcher toute redescente du piston (100) par pression sur la tige d'actionnement (120) sauf à désengager la première crémaillère (122) par rotation du piston (100) dans le corps de seringue (20) autour de l'axe longitudinal (A1) pour amener la première crémaillère (122) en regard de la première surface vierge (22) de la paroi interne (21) du corps de seringue (20), et
dans laquelle la tête de piston (110) comporte :
- une partie terminale (114) faisant face à l'extrémité inférieure (20A) du corps de seringue (20), la partie terminale (114) étant telle qu'elle empêche tout désengagement complet du piston (100) du corps de seringue (20) par blocage par la ou les butées, et
- une embase (111), raccordée à la partie terminale (114), comprenant à sa périphérie au moins une première encoche (112) et au moins une première rainure longitudinale (115),
la première encoche (112) et la première rainure longitudinale (115) de l'embase (111) étant conformées et constituées pour permettre la remontée et le maintien du piston (100) en position haute lorsque la première encoche (112) est en regard avec la première butée (32), et empêcher toute redescente du piston (100) le long du corps de seringue (20), sauf à désengager la tête de piston (110) par rotation du piston (100) dans le corps de seringue (20) autour de l'axe longitudinal (A1) pour amener la première rainure longitudinale (115) en regard de la première butée (32).

2. Seringue (10) selon la revendication 1, dans laquelle :
- le corps de seringue (20) comporte une deuxième butée (33) semblable à la première butée (32), et située le long de la paroi interne (21) du corps de seringue (20) à proximité de l'ouverture supérieure (20C), de sorte que la première et la deuxième butées (32, 33) forment entre elles une première et une deuxième surfaces vierges (22, 23) sur la paroi interne (21) du corps de seringue (20), et dans laquelle
- la tige d'actionnement (120) comporte sur sa surface externe (121) une deuxième crémaillère (123) semblable à la première crémaillère (122) et disposée longitudinalement le long de la tige d'actionnement (120), de sorte que, lorsque la première crémaillère (122) est en regard de la première butée (32), la deuxième crémaillère (123) est en regard de la deuxième butée (33).

3. Seringue (10) selon la revendication 2, dans laquelle :
- les première et deuxième butées (32, 33) sont sensiblement diamétralement opposées, et
- les première et deuxième crémaillères (122, 123) sont également sensiblement diamétralement opposées.

4. Seringue (10) selon l'une quelconque des revendications 1 à 3, dans laquelle l'embase (111) comprend à sa périphérie :
- une deuxième encoche (113) semblable à la première encoche (112), et
- une deuxième rainure longitudinale (116) semblable à la première rainure longitudinale (115), disposée de telle sorte que, lorsque la première rainure longitudinale (115) est en regard de la première butée (32), la deuxième rainure longitudinale (116) est en regard de la deuxième butée (33).

5. Seringue (10) selon la revendication 4, dans laquelle les première et deuxième rainures longitudinales (115, 116) sont sensiblement diamétralement opposées.

6. Seringue (10) selon l'une quelconque des revendications 1 à 5, dans laquelle la tige d'actionnement (120) du piston est détachable de la tête de piston (110).

7. Seringue (10) selon la revendication 6, dans laquelle :
- la tête de piston (110) comporte un logement de réception (130) solidaire de l'embase (111), de sorte que, lorsque le piston (100) est en position haute, le logement de réception (130) se trouve hors du corps de seringue (20), et dans laquelle
- la tige d'actionnement (120) comporte à l'une de ses extrémités un élément de raccordement (124) à la tête de piston (110), l'élément de raccordement (124) présentant une forme complémentaire du logement de réception (130) de la tête de piston (110), de sorte que, lorsque le piston (100) est en position haute, l'élément de raccordement (124) peut s'engager ou se désengager du logement de réception (130) et que, lorsque l'élément de raccordement (124) est engagé à l'intérieur du logement de réception (130), la tige d'actionnement (120) et la tête de piston (110) sont solidaires en translation selon l'axe longitudinal (A1) et en rotation autour de l'axe longitudinal (A1).

8. Seringue (10) selon la revendication 7, dans laquelle le logement de réception (130) comprend une face latérale ouverte (131) et une face supérieure (132) présentant une ouverture centrale (133) sensiblement positionnée selon l'axe longitudinal (A1) de la seringue (10), de telle sorte que l'élément de raccordement (124) s'engage ou se désengage du logement de réception (130) par glissement au travers de la face latérale ouverte (131), l'ouverture centrale (133) permettant le passage de la tige d'actionnement (120).

## Patentansprüche

1. Spritze (10) mit
- einem Spritzenkörper (20), der sich entlang einer Längsachse (A1) von einem unteren Ende (20A) bis zu einem oberen Ende (20B) erstreckt, wobei das untere Ende (20A) des Spritzenkörpers (20) das Befestigen einer Nadel oder einer Kanüle ermöglicht und das obere Ende (20B) des Spritzenkörpers (20) eine obere Öffnung (20C) aufweist, die das Einführen eines Kolbens in den Spritzenkörper (20) ermöglicht, wobei der Spritzenkörper (20) eine innere Wand (21) aufweist und wenigstens einen ersten Anschlag (32) aufweist, der entlang der inneren Wand (21) des Spritzenkörpers (20) in der Nähe der oberen Öffnung (20C) angeordnet ist, wobei der erste Anschlag (32) dazu ausgelegt ist, wenigstens eine freie Fläche (22) auf der inneren Wand (21) des Spritzenkörpers (20) zu lassen, und
- einem Kolben (100), der entlang der inneren Wand (21) des Spritzenkörpers (20) entlang der Längsachse (A1) gleitet, wobei der Kolben (100) einen Kolbenkopf (110) und eine mit dem oberen Ende (110B) des Kolbenkopfes (110) verbundene Betätigungsstange (120) aufweist, wobei das obere Ende (110B) des Kolbenkopfes (110) zur oberen Öffnung (20C) des Spritzenkörpers (20) hin gerichtet ist, wobei die Betätigungsstange (120) des Kolbens (100) auf ihrer äußeren Oberfläche (121) wenigstens eine in Längsrichtung entlang der Betätigungsstange (120) angeordnete erste Zahnstange (122) aufweist, wobei die erste Zahnstange (122) nach außen vorstehende Rastnasen (122A) aufweist,
wobei der erste Anschlag (32) und die nach außen vorstehenden Rastnasen (122A) so ausgelegt und dafür bestimmt sind, beim Hochziehen des Kolbens (100) im Spritzenkörper (20) von einer unteren Position, in der sich der Kolbenkopf (110) in der Nähe des unteren Endes (20A) des Spritzenkörpers (20) befindet, zu einer oberen Position, in der sich der Kolbenkopf (110) in der Nähe des oberen Endes (20B) des Spritzenkörpers (20) befindet, wenn der erste Anschlag (32) und die erste Zahnstange (122) einander gegenüberstehen, dem Kolben (100) ein Hochgehen von Nase zu Nase entlang des Spritzenkörpers (20) zu ermöglichen,
wobei die Spritze (10) **dadurch gekennzeichnet ist, daß** der erste Anschlag (32) und die nach außen vorstehenden Rastnasen (122A) außerdem so ausgelegt und dafür bestimmt sind, jegliches erneute Hinabgehen des Kolbens (100) durch Druck auf die Betätigungsstange (120) zu verhindern, außer wenn die erste Zahnstange (122) durch Drehung des Kolbens (100) im Spritzenkörper (20) um die Längsachse (A1) freigesetzt wird, um die erste Zahnstange (122) vor die erste freie Fläche (22) der inneren Wand (21) des Spritzenkörpers (20) zu bringen,
und bei der der Kolbenkopf (110)
- ein Endstück (114), das dem unteren Ende (20A) des Spritzenkörpers (20) zugewandt ist, wobei das Endstück (114) so ausgelegt ist, daß es jegliches vollständige Herausgehen des Kolbens (100) aus dem Spritzenkörper (20) durch Blockieren durch den oder die Anschläge verhindert, und
- einen mit dem Endstück (114) verbundenen Sockel (111), der an seinem Umfang wenigstens eine erste Kerbe (112) und wenigstens eine erste Längsrille (115) aufweist,
aufweist,
wobei die erste Kerbe (112) und die erste Längsrille (115) des Sockels (111) so ausgelegt und dafür bestimmt sind, das Hochgehen des Kolbens (100) und das Halten desselben in der oberen Position zu ermöglichen, wenn die erste Kerbe (112) dem ersten Anschlag (32) gegenübersteht, und jegliches erneute Hinabgehen des Kolbens (100) entlang des Spritzenkörpers (20) zu verhindern, außer wenn der Kolbenkopf (110) durch Drehung des Kolbens (100) im Spritzenkörper (20) um die Längsachse (A1) freigesetzt wird, um die erste Längsrille (115) vor den ersten Anschlag (32) zu bringen.

2. Spritze (10) gemäß Anspruch 1, bei der
- der Spritzenkörper (20) einen zweiten Anschlag (33) aufweist, der dem ersten Anschlag (32) ähnlich ist und entlang der inneren Wand (21) des Spritzenkörpers (20) in der Nähe der oberen Öffnung (20C) angeordnet ist, so daß der erste und der zweite Anschlag (32, 33) zwischen sich auf der inneren Wand (21) des Spritzenkörpers (20) eine erste und eine zweite freie Fläche (22, 23) bilden, und bei der
- die Betätigungsstange (120) auf ihrer äußeren Oberfläche (121) eine zweite Zahnstange (123) aufweist, die der ersten Zahnstange (122) ähnlich ist und in Längsrichtung entlang der Betätigungsstange (120) angeordnet ist, so daß dann, wenn die erste Zahnstange (122) dem ersten Anschlag (32) gegenübersteht, die zweite Zahnstange (123) dem zweiten Anschlag (33) gegenübersteht.

3. Spritze (10) gemäß Anspruch 2, bei der
- der sich erste und der zweite Anschlag (32, 33) im Wesentlichen diametral gegenüberstehen und
- die sich erste und die zweite Zahnstange (122, 123) ebenfalls im Wesentlichen diametral gegenüberstehen.

4. Spritze (10) gemäß einem der Ansprüche 1 bis 3, bei der der Sockel (111) an seinem Umfang
- eine der ersten Kerbe (112) ähnliche zweite Kerbe (113) und
- eine der ersten Längsrille (115) ähnliche zweite Längsrille (116) aufweist, die so angeordnet ist, daß dann, wenn die erste Längsrille (115) dem ersten Anschlag (32) gegenübersteht, die zweite Längsrille (116) dem zweiten Anschlag (33) gegenübersteht.

5. Spritze (10) gemäß Anspruch 4, bei der sich die erste und die zweite Längsrille (115, 116) im Wesentlichen diametral gegenüberstehen.

6. Spritze (10) gemäß einem der Ansprüche 1 bis 5, bei der die Betätigungsstange (120) des Kolbens vom Kolbenkopf (110) abnehmbar ist.

7. Spritze (10) gemäß Anspruch 6, bei der
- der Kolbenkopf (110) einen mit dem Sockel (111) fest verbundenen Aufnahmeraum (130) derart aufweist, daß dann, wenn sich der Kolben (100) in der oberen Position befindet, sich der Aufnahmeraum (130) außerhalb des Spritzenkörpers (20) befindet, und bei der
- die Betätigungsstange (120) an einem ihrer Enden ein Element (124) für den Anschluß am Kolbenkopf (110) aufweist, wobei das Anschlußelement (124) eine zum Aufnahmeraum (130) des Kolbenkopf (110) komplementäre Form aufweist, so daß dann, wenn sich der Kolben (100) in der oberen Position befndet, das Anschlußelement (124) in den Aufnahmeraum (130) eingesetzt oder aus diesem herausgenommen werden kann, und daß dann, wenn das Anschlußelement (124) in den Aufnahmeraum (130) eingesetzt ist, die Betätigungsstange (120) und der Kolbenkopf (110) in der Richtung der Längsachse (A1) translationsfest und um zum Drehen um die Längsachse (A1) drehfest miteinander verbunden sind.

8. Spritze (10) gemäß Anspruch 7, bei der der Aufnahmeraum (130) eine offene seitliche Seite (131) und eine Oberseite (132) mit einer zentralen Öffnung (133), die im Wesentlichen auf der Längsachse (A1) der Spritze (10) liegt, derart aufweist, daß das Anschlußelement (124) durch Gleiten durch die offene seitliche Seite (131) in den Aufhahmeraum (130) eingesetzt oder aus diesem herausgenommen wird, wobei die zentrale Öffnung (133) das Hindurchführen der Betätigungsstange (120) ermöglicht.

## Claims

1. A syringe (10) including:
- a syringe body (20) extending along a longitudinal axis (A1) from a lower end (20A) to an upper end (20B), the lower end (20A) of the syringe body (20) allowing the fixation of a needle or a cannula and the upper end (20B) of the syringe body (20) having an upper opening (20C) allowing the introduction of a plunger into the syringe body (20), the syringe body (20) having an inner wall (21) and including at least one first stop (32) located along the inner wall (21) of the syringe body (20) near the upper opening (20C), the first stop (32) being adapted to leave at least one first blank surface (22) on the inner wall (21) of the syringe body (20), and
- a plunger (100) sliding along the inner wall (21) of the syringe body (20) along the longitudinal axis (A1), the plunger (100) comprising a plunger head (110) and an operating rod (120) connected to the upper end (110B) of the plunger head (110), the upper end (110B) of the plunger head (110) being directed towards the upper opening (20C) of the syringe body (20), the operating rod (120) of the plunger (100) including, on its outer surface (121), at least one first rack (122) arranged longitudinally along the operating rod (120), the first rack (122) comprising protruding teeth (122A),
the first stop (32) and the protruding teeth (122A) being shaped and constituted so as, during the upward movement of the plunger (100) in the syringe body (20) from a low position where the plunger head (110) is near the lower end (20A) of the syringe body (20) to a high position where the plunger head (110) is near the upper end (20B) of the syringe body (20), when the first stop (32) and the first rack (122) are opposite to each other, to allow a tooth-by-tooth upward movement of the plunger (100) along the syringe body (20),
the syringe (10) being **characterized in that** the first stop (32) and the protruding teeth (122A) are also shaped and constituted so as to prevent any downward movement of the plunger (100) under the effect of a push on the operating rod (120), but to disengage the first rack (122) by rotation of the plunger (100) in the syringe body (20) about the longitudinal axis (A1) to bring the first rack (122) opposite the first blank surface (22) of the inner wall (21) of the syringe body (20), and,
wherein the plunger head (110) includes:
- a terminal part (114) facing the lower end (20A) of the syringe body (20), the terminal part (114) being such that it prevents any full disengagement of the plunger (100) from the syringe body (20) thanks to a locking by the stop(s), and
- a base (111), connected to the terminal part (114), comprising at its periphery at least one first notch (112) and at least one first longitudinal groove (115),
the first notch (112) and the first longitudinal groove (115) of the base (111) being shaped and constituted so as to allow the upward movement and the holding of the plunger (100) in the high position when the first groove (112) is opposite the first stop (32), and to prevent any downward movement of the plunger (100) along the syringe body (20), but to disengage the plunger head (110) by rotation of the plunger (100) in the syringe body (20) about the longitudinal axis (A1) to bring the first longitudinal groove (115) opposite the first stop (32).

2. The syringe (10) according to claim 1, wherein:
- the syringe body (20) includes a second stop (33) similar to the first stop (32), and located along the inner wall (21) of the syringe body (20), near the upper opening (20C), so that the first and the second stops (32, 33) form between each other a first and a second blank surfaces (22, 23) on the inner wall (21) of the syringe body (20), and wherein
- the operating rod (120) includes on its outer surface (121) a second rack (123) similar to the first rack (122), and arranged longitudinally along the operating rod (120), so that, when the first rack (122) is opposite the first stop (32), the second rack (123) is opposite the second stop (33).

3. The syringe (10) according to claim 2, wherein:
- the first and second stops (32, 33) are substantially diametrically opposed to each other, and
- the first and second racks (122, 123) are also substantially diametrically opposed.

4. The syringe (10) according to any one of claims 1 to 3, wherein the base (111) comprises at its periphery:
- a second notch (113) similar to the first notch (112), and
- a second longitudinal groove (116) similar to the first longitudinal groove (115), arranged in such a manner that, when the first longitudinal groove (115) is opposite the first stop (32), the second longitudinal groove (116) is opposite the second stop (33).

5. The syringe (10) according to claim 4, wherein the first and second longitudinal grooves (115, 116) are substantially diametrically opposed to each other.

6. The syringe (10) according to any one of claims 1 to 5, wherein the operating rod (120) of the plunger is detachable from the plunger head (110).

7. The syringe (10) according to claim 6, wherein:
- the plunger head (110) includes a receiving recess (130) integral with the base (111) so that, when the plunger (100) is in the high position, the receiving recess (130) is outside the syringe body (20), and wherein
- the operating rod (120) includes at one of its ends an element (124) for connection to the plunger head (110), the connection element (124) having a shape that is complementary of that of the receiving recess (130) of the piston head (110), so that, when the plunger (100) is in the high position, the connection element (124) can be engaged into or disengaged from the receiving recess (130) and that, when the connection element (124) is engaged into the receiving recess (130), the operation rod (120) and the plunger head (110) are translationally integral with each other along the longitudinal axis (A1) and rotationally integral with each other about the longitudinal axis (A1).

8. The syringe (10) according to claim 7, wherein the receiving recess (130) comprises an open lateral face (131) and an upper face (132) having a central opening (133) substantially positioned along the longitudinal axis (A1) of the syringe (10), so that the connection element (124) is engaged into or disengaged from the receiving recess (130) by being slid through the open lateral face (131), the central opening (133) allowing the passage of the operating rod (120).
